**Europäisches Patentamt**

**European Patent Office**

**Office européen des brevets**

(19)

(11) Veröffentlichungsnummer : **0 446 559 B1**

(12) ## EUROPÄISCHE PATENTSCHRIFT

(45) Veröffentlichungstag der Patentschrift :
**01.09.93 Patentblatt 93/35**

(51) Int. Cl.$^5$ : **A61F 2/30**

(21) Anmeldenummer : **90810957.2**

(22) Anmeldetag : **06.12.90**

(54) **Einzementierbare Hüftgelenkspfanne aus Kunststoff.**

(30) Priorität : **13.03.90 CH 799/90**

(43) Veröffentlichungstag der Anmeldung :
**18.09.91 Patentblatt 91/38**

(45) Bekanntmachung des Hinweises auf die
Patenterteilung :
**01.09.93 Patentblatt 93/35**

(84) Benannte Vertragsstaaten :
**AT CH DE FR GB IT LI**

(56) Entgegenhaltungen :
**CH-A- 649 913
FR-A- 2 134 170
FR-A- 2 250 279
US-A- 3 740 769
US-A- 3 806 960**

(73) Patentinhaber : **GEBRÜDER SULZER
AKTIENGESELLSCHAFT
Zürcherstrasse 9
CH-8401 Winterthur (CH)**

(72) Erfinder : **Willert, Hans-Georg, Prof. Dr.med.
Schlegelweg 9
W-3400 Göttingen (DE)**
Erfinder : **Koch, Rudolf
Oberdorfstrasse 229
CH-8267 Berlingen (CH)**
Erfinder : **Bürgi, Maya
Chaennerwisstrasse 5
CH-8352 Räterschen (CH)**

**Beschreibung**

Die Erfindung betrifft eine einzementierbare Hüftgelenkspfanne, die aus einer Schale mit einer Halbkugelinnenform besteht. Die Verankerung von einem allseitig abgerundeten Schaft einer Hüftgelenksprothese mit Hilfe von Knochenzement ist in der CH-A-666 611 mit Vertiefungen als Verankerungsstrukturen gezeigt. Zementfreie Hüftgelenkspfannen mit einer von Vertiefungen durchsetzten Oberfläche zum Einwachsenlassen von Knochengewebe sind in der US-A-3,740,769 aufgeführt. In der US-A-3,806,960 wird eine einzementierbare Hüftgelenkspfanne gemäß dem Oberbegriff des Anspruchs 1 gezeigt, die aus einer Metallschale besteht, welche auf ihrer Aussenseite mit hochelastischem Silikongummi beschichtet ist, wobei im Gummi Vorsprünge und Einbuchtungen angebracht sind.

Ein Problem bei der Verankerung einer Hüftgelenkspfanne mit Knochenzement besteht darin, dass die äussere Schalenfläche mit einer halbkugelähnlichen Form nur begrenzte Verankerungsmöglichkeiten für einen auf Zugspannungen empfindlichen Knochenzement bietet.

Hier schafft die Erfindung Abhilfe. Sie löst die Aufgabe, eine gute Verankerung zwischen einer Hüftgelenkspfanne, die aus einem gegenüber dem Gelenkkopf verschleissarmen Kunststoff besteht, und Knochenzement herzustellen. Sie löst diese Aufgabe dadurch, dass sie aus formbeständigem Kunsstoff besteht und auf der Aussenform regelmässige sich nach aussen konisch öffnende Vertiefungen aufweist, die ein annähernd festes Verhältnis zwischen Tiefe und Weite besitzen und die vom Rand der Schale zum Pol hin in Volumen, Tiefe und Weite abnehmend sind.

Die Vorteile der Erfindung sind darin zu sehen, dass die beschriebenen Vertiefungen den Knochenzement in Formen aushärten lassen, die eine grosse Kraftübertragung der ganzen Pfanne mit geringen Spannungsspitzen im Knochenzement ermöglichen. Die abhängigen Ansprüche 2 bis 9 beziehen sich auf vorteilhafte Weiterbildungen der Erfindung.

Im folgenden wird die Erfindung an einem Ausführungsbeispiel beschrieben. Es zeigen:

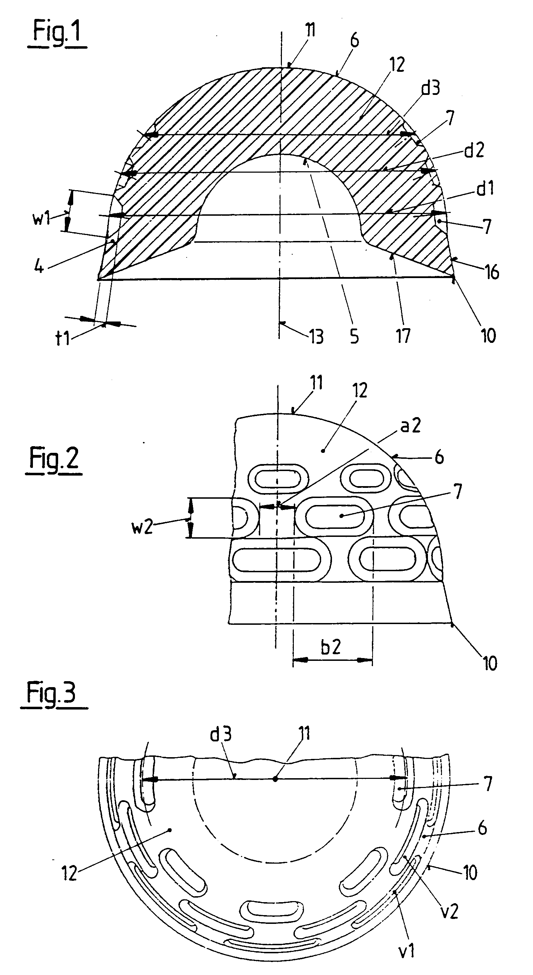

Fig. 1     Einen Schnitt durch eine Hüftgelenkspfanne längs ihrer Schalenachse mit konisch sich nach aussen öffnenden Vertiefungen,

Fig. 2     den Ausschnitt einer Seitenansicht der Hüftgelenkspfanne gemäss Fig. 1 und

Fig. 3     den Ausschnitt einer Draufsicht der Hüftgelenkspfanne gemäss Fig. 1.

In den Figuren ist eine einzementierbare Hüftgelenkspfanne 4 aus formbeständigem Kunststoff, wie Polyethylen, gezeigt, die aus einer Schale 12 mit einer Halbkugelinnenform 5 und mit einer halbkugelähnlichen Aussenform 6 besteht, wobei diese als Innenkonus 17 und als Aussenkonus 16 zum Schalenrand 10 auslaufen. Auf verschiedenen zur Schalenachse 13 senkrechten Durchmessern d1, d2, d3 der Aussenform 6 sind regelmässige, sich nach aussen konisch öffnende Vertiefungen 7 angebracht, die in sich ein festes Verhältnis $\beta$ von ihrer radialen Tiefe t zur tangentialen Weite w aufweisen, wobei $\beta$ einem Wert zwischen 0,1 und 0,5 entspricht, z.B. $\frac{t1}{w1} = 0{,}2$. Die Dimensionen Tiefe t, Weite w und Volumen v der Vertiefungen sind in Abhängigkeit von den zur Schalenachse 13 senkrechten Durchmessern d1, d2, d3 vom Schalenrand 10 zum Pol 11 hin abnehmend. Die Tiefe t und die Weite w zweier Vertiefungen mit unterschiedlichen, zur Schalenachse 13 senkrechten Durchmesserabständen d1 und d2 verhalten sich wie das mit einem Exponenten $\alpha$ versehene Verhältnis ihrer zur Schalenachse 13 senkrechten Durchmesserabstände d2, d1, wobei der Exponent $\alpha$ einen Wert zwischen 1,2 und 1,6 aufweist, z.B. $\frac{w2}{w1} = \left(\frac{d2}{d1}\right)^{1,4}$. Entsprechend nehmen die Volumen der Vertiefungen mit einem Exponenten $\delta$ für das Durchmesserverhältnis d2/d1 vom Rand zum Pol 11 hin ab, wobei $\delta$ einen Wert zwischen 3,4 und 4,2 annimmt, z.B. $\frac{V2}{V1} = \left(\frac{d2}{d1}\right)^{3,8}$.

Die auf einem zur Schalenachse 13 senkrechten Durchmesserabstand d liegenden Vertiefungen 7 haben gleiche Dimensionen und weisen über den gemeinsamen Schalenumfang einen kleineren Abstand a zueinander auf als ihre Breite b beträgt, z.B. a2 < b2. Die in einer Ebene liegenden Vertiefungen mit gleichem Durchmesserabstand sind am Umfang zu den Vertiefungen 7 der direkt benachbarten Ebenen versetzt angeordnet. Die Anzahl der Vertiefungen ist gleich pro Ebene mit Durchmesserabstand d1, d2, d3.

Die Auswahl der die Vertiefungen beschreibenden Exponenten und Faktoren wurde so getroffen, dass einerseits mit der Belastung der Hüftgelenkspfanne keine zu grossen Zugspannungen im Knochenzement auftreten und andererseits alle Vertiefungen beim Einsetzen der Hüftgelenksschale vollständig mit Knochenzement gefüllt werden, bevor dieser aushärtet.

**Patentansprüche**

1. Einzementierbare Hüftgelenkpfanne (4) bestehend aus einer Schale (12) mit einer Halbkugelinnenform (5), dadurch gekennzeichnet, dass die Hüftgelenkpfanne (4) aus formbeständigem Kunststoff besteht und auf der Aussenform (6) regelmässige sich nach aussen konisch öffnende Vertiefungen (7) aufweist, die ein annähernd festes Verhältnis zwischen Tiefe (t) und Weite (w) besitzen und die vom Rand (10) der Schale (12) zum Pol (11) hin in Volumen (v), Tiefe (t) und Weite (w) abnehmend sind.

2. Hüftgelenkpfanne nach Anspruch 1, dadurch gekennzeichnet, dass die Schale (12) aus Polyäthylen besteht.

3. Hüftgelenkpfanne nach Anspruch 1 oder 2, dadurch gekennzeichnet, dass das jeweilige Verhältnis von Tiefe (t) zu Weite (w) der Vertiefungen (7) einem Faktor $\beta$ zwischen 0,1 und 0,5 entspricht.

4. Hüftgelenkpfanne nach einem der Ansprüche 1 bis 3, dadurch gekennzeichnet, dass die zum Pol (11) hin liegenden Vertiefungen (7) im Vergleich zu einer am Rand liegenden Vertiefung (7) mit ihrer Weite (w) und Tiefe (t) im Verhältnis ihrer zur Schalenachse (13) senkrechten Durchmesserabstände (d) mit einem Exponenten $\alpha$, der einen Wert von 1,2 bis 1,6 aufweist, abnehmen.

5. Hüftgelenkpfanne nach einem der Ansprüche 1 bis 4, dadurch gekennzeichnet, dass die zum Pol hin liegenden Vertiefungen (7) im Vergleich zu einer am Rand liegenden Vertiefung mit ihrem Volumen (v) im Verhältnis ihrer zur Schalenachse (13) senkrechten Durchmesserabstände (d) mit einem Exponenten $\delta$, der einen Wert von 3,4 bis 4,2 aufweist, abnehmen.

6. Hüftgelenkpfanne nach einem der Ansprüche 1 bis 5, dadurch gekennzeichnet, dass mehrere Vertiefungen (7) jeweils auf einem senkrecht zur Schalenachse (13) stehenden Schnitt der Aussenform (6) liegen und gleichmässig über dessen Umfang verteilt sind.

7. Hüftgelenkpfanne nach einem der Ansprüche 1 bis 6, dadurch gekennzeichnet, dass die Anzahl der auf verschiedenen Durchmesserabständen (d1,d2,d3) liegenden Vertiefungen (7) gleich gross ist.

8. Hüftgelenkpfanne nach einem der Ansprüche 1 bis 7, dadurch gekennzeichnet, dass die Vertiefungen (7) eines Schnittes mit Durchmesserabstand (d2) zu den Vertiefungen (7) eines benachbarten Durchmesserabstandes (d1,d3) am Umfang versetzt sind.

9. Hüftgelenkpfanne nach einem der Ansprüche 1 bis 8, dadurch gekennzeichnet, dass die Abstände (a) zwischen den Vertiefungen (7) am Umfang eines Durchmesserabstandes (d1,d2,d3) kleiner sind als die am gleichen Umfang gemessene Breite (b) der Vertiefungen (7).


**Claims**

1. An acetabulum (4) which can be cemented into position, consisting of a shell (12) having a hemispherical inner contour (5),
   **characterised in that** the acetabulum (4) is made from non-deformable plastic and on the outer contour (6) comprises regular cavities (7) opening conically towards the outside, which have a roughly fixed ratio between depth (t) and width (w) and which from the edge (10) of the shell (12) to the peak (11) decrease in volume (v), depth (t) and width (w).

2. An acetabulum according to Claim 1,
   **characterised in that** the shell (12) is made from polyethylene.

3. An acetabulum according to Claim 1 or 2,
   **characterised in that** the respective ratio of depth (t) to width (w) of the cavities (7) corresponds to a factor $\beta$ of between 0.1 and 0.5.

4. An acetabulum according to one of Claims 1 to 3,
   **characterised in that** the width (w) and depth (t) of the cavities (7) lying towards the peak (11) decrease when compared with a cavity (7) lying on the edge in the ratio of their diameter spacings (d) at right angles to the shell axis (13) with an exponent $\alpha$, which has a value of 1.2 to 1.6.

5. An acetabulum according to one of Claims 1 to 4,
**characterised in that** the volume (v) of the cavities (7) lying towards the peak decreases in comparison with the volume of a cavity lying on the edge in the ratio of their diameter spacings (d) at right angles to the shell axis (13) with an exponent $\delta$, which has a value of 3.4 to 4.2.

6. An acetabulum according to one of Claims 1 to 5,
**characterised in that** several cavities (7) are positioned respectively on a section of the outer contour (6) which is at right angles to the shell axis (13) and are distributed regularly over its periphery.

7. An acetabulum according to one of Claims 1 to 6,
**characterised in that** the number of the cavities (7) lying on different diameter spacings (d1, d2, d3) is equal.

8. An acetabulum according to one of Claims 1 to 7,
**characterised in that** the cavities (7) of a section with diameter spacing (d2) are staggered over the periphery with respect to the cavities (7) of an adjacent diameter spacing (d1, d3).

9. An acetabulum according to one of Claims 1 to 8,
**characterised in that** the spacings (a) between the cavities (7) on the periphery of a diameter spacing (d1, d2, d3) are smaller than the breadth (b) of the cavities (7) measured on the same periphery.

**Revendications**

1. Cotyle prothétique (4) à fixation par ciment composé d'une coque (12) ayant une forme intérieure hémisphérique (5), caractérisé en ce que le cotyle prothétique (4) consiste en une matière plastique stable et présente sur la forme extérieure (6) des cavités régulières coniques (7) s'ouvrant vers l'extérieur, qui possèdent un rapport quasiment constant entre la profondeur (t) et l'ouverture (w) et qui diminuent en volume (v), profondeur (t) et ouverture (w) du bord (10) de la coque (12) vers le pôle (11).

2. Cotyle prothétique suivant la revendication 1, caractérisé en ce que la coque (12) est réalisée en polyéthylène.

3. Cotyle prothétique suivant la revendication 1 ou 2, caractérisé en ce que le rapport correspondant de la profondeur (t) à l'ouverture (w) des cavités (7) correspond à un facteur $\beta$ compris entre 0,1 et 0,5.

4. Cotyle prothétique suivant l'une des revendications 1 à 3, caractérisé en ce que les cavités (7) se rapprochant du pôle (11), en comparaison avec une cavité (7) située au bord, diminuent en ouverture (w) et profondeur (t) proportionnellement à la puissance a du rapport de leurs diamètres (d) perpendiculaires à l'axe (13) de la coque, l'exposant $\alpha$ présentant une valeur comprise entre 1,2 et 1,6.

5. Cotyle prothétique suivant l'une des revendications 1 à 4, caractérisé en ce que les cavités (7) se rapprochant du pôle, en comparaison avec une cavité située au bord, diminuent de volume (v) proportionnellement à la puissance $\delta$ du rapport de leurs diamètres (d) perpendiculaires à l'axe (13) de la coque, l'exposant $\delta$ présentant une valeur comprise entre 3,4 et 4,2.

6. Cotyle prothétique suivant l'une des revendications 1 à 5, caractérisé en ce que plusieurs cavités (7) se situent chacune sur une section de la forme extérieure (6), perpendiculaire à l'axe (13) de la coque, et sont réparties de façon régulière sur sa circonférence.

7. Cotyle prothétique suivant l'une des revendications 1 à 6, caractérisé en ce que le nombre des cavités (7) situées sur différents diamètres (d1, d2, d3) est identique.

8. Cotyle prothétique suivant l'une des revendications 1 à 7, caractérisé en ce que les cavités (7) d'une section de diamètre (d2) sont décalées sur la circonférence par rapport aux cavités (7) d'un diamètre voisin (d1, d3).

9. Cotyle prothétique suivant l'une des revendications 1 à 8, caractérisé en ce que les distances (a) entre les cavités (7) sur la circonférence d'un diamètre (d1, d2, d3) sont inférieures à la largeur (b) des cavités (7), mesurée sur la même circonférence.

<u>Fig.1</u>

<u>Fig.2</u>

<u>Fig.3</u>